# EUROPEAN PATENT APPLICATION

(11) **EP 4 134 907 A1**
(43) Date of publication of application: **15.02.2023**
(21) Application number: 21190285.3
(22) Date of filing: 09.08.2021
(51) Int. Cl.: G06T 7/00, A61B 6/00

(54) **HETEROGENEITY ANALYSIS IN 3D X-RAY DARK-FIELD IMAGING**

(71) Applicant: Koninklijke Philips N.V., 5656 AG Eindhoven (NL)
(72) Inventor: BRAUNAGEL, Andriy Yaroshenko, 5656 AE Eindhoven (NL); KOEHLER, Thomas, 5656 AE Eindhoven (NL)
(74) Representative: Philips Intellectual Property & Standards

(57) **Abstract**

Apart from signal changes in three-dimensional X-ray dark-field (3D-DAX) imaging, also significant signal heterogeneity can be revealed. A method and apparatus for heterogeneity analysis in 3D X-ray dark field imaging are provided, to enable the generation of parameters for an objective quantification and assessment of signal heterogeneity in 3D-DAX image data of a subject. In addition, a system for 3D X-ray imaging comprising said apparatus, a computer program element for carrying out the method and/or controlling the apparatus and/or system, and a computer readable medium having stored thereon the program element, are provided.

## Description

### FIELD OF THE INVENTION

The invention relates to the field of 3D X-ray dark-field imaging. The invention relates to a method and apparatus for heterogeneity analysis in 3D X-ray dark-field imaging, a system for 3D X-ray imaging comprising said apparatus, a computer program element and a computer readable medium.

### BACKGROUND OF THE INVENTION

Phase contrast X-ray imaging (PCI) offers greater sensitivity with respect to conventional X-ray imaging, enabling imaging of low-density materials non-destructively and hence offering an advantage in clinical applications such as mammography, lung imaging, bone imaging, etc.

A single phase contrast X-ray imaging system, regardless of its implementation, offers both phase contrast (PC) and dark-field (DAX) information (in addition to attenuation/transmission X-ray information). In more detail, comparison of an interference pattern with and without an object in the x-ray beam allows to extract perfectly registered (since originating from the same dataset) conventional X-ray attenuation, DAX, and PC signals. The perfect spatial registration of the signals in projection domain directly translates to perfect registration in image domain after tomographic reconstruction and implies the possibility of signal combination for an increased diagnostic accuracy.

Recent studies demonstrated that DAX can be used in order to precisely depict alveolar changes, earlier than this is possible with conventional attenuation-based X-ray imaging. This is of advantage for an improved diagnostic accuracy for the diagnosis and staging of numerous pulmonary disorders, such as emphysema, chronic obstructive pulmonary disease (COPD), lung fibrosis, pneumonia and lung cancer.

Measurable indicators of the severity or disease state through the DAX signal analysis would be useful for an objective measurement of the progress of the disease, to evaluate therapies, etc. US 2018/0271465 A1 has contributed to providing diagnostic information by generating a lung condition map based on two-dimensional (2D) X-ray images. The generation of a lung condition map based on projection images requires an estimation for the depth of the imaged lung depth by image processing and a normalization of the DAX signal with spatially corresponding data values of the lung depth map so that the DAX signal comes out normalized to the lung thickness. Instead, with three-dimensional (3D) X-ray imaging information, such as that provided by computed tomography, X-ray tomosynthesis or C-arm X-ray imaging, the mentioned procedures are not necessary, and therefore, could be potentially used for an even more precise evaluation and quantification of a disorder severity. However, there are no defined clinical biomarkers for disease diagnosis based on the analysis of 3D-DAX data.

### SUMMARY OF THE INVENTION

It would be advantageous to have an improved technique for providing diagnostic information based on 3D X-ray imaging data.

Preclinical data led to an insight of the inventors that apart from signal changes in three-dimensional X-ray dark-field (3D-DAX) imaging, also significant signal inhomogeneities, i.e. signal heterogeneity, can be revealed. The inventors have recognized that this may be leveraged to generate a clinical parameter that may act as clinical biomarker to help physicians in their assessments, e.g., to determine a severity of the disease and/or localization of problematic areas. The physician may then take this information into account to select the best therapy option. Towards this end, in a first aspect of the invention, a method for heterogeneity analysis in 3D-DAX imaging is provided.

The method for heterogeneity analysis comprises: receiving 3D X-ray dark-field field (DAX) image data and/or 3D X-ray attenuation image data of a subject; segmenting the X-ray attenuation image data and/or the DAX image data to identify a region of interest (ROI) in the DAX image data; analyzing the ROI image data heterogeneity by performing a statistical analysis of the ROI in the DAX image data based on dividing the ROI in the DAX image data in a number N of subregions with an associated subregion size (*SRₘₑₐₛ*), and quantifying a corresponding measure of image data heterogeneity (*Dₘₑₐₛ*); and providing an indicator of image data heterogeneity.

In this way, the heterogeneity in 3D-DAX imaging is analyzed and hence provides the means to generate an objective measure (e.g., a clinical parameter) of signal heterogeneity in a ROI which can be further assessed. An objective measure represents a more reliable source of information available to the user and allows more reliable comparison between measurements taken at different times, of different patients and/or on different 3D-DAX imaging devices.

In some embodiments, quantifying the measure of image data heterogeneity comprises obtaining a measure of central tendency of the DAX image data per subregion *N*, and a measure of statistical dispersion based on the ensemble of measures of central tendency of the DAX image data per subregion *N*. By obtaining a measure of central tendency within each subregion, it is ensured that the statistical dispersion analysis is done on the proper length scale as defined by the subregion size (*SRₘₑₐₛ*)*.*

In some embodiments, the indicator of image data heterogeneity comprises the measure of image data heterogeneity (*Dₘₑₐₛ*) and/or the corresponding subregion size (*SRₘₑₐₛ*)*.* These parameters provide an objective way to characterize the heterogeneity of a ROI.

In an embodiment the ROI may be an ensemble of ROIs (*ROIᵢ, i=[1,n]*), each ROI characterized by a measure of image data heterogeneity for a subregion size, and wherein the indicator of image data heterogeneity is an ensemble of measures of image data heterogeneity (*D_{meas,i},i=[1,n]*) and/or an ensemble of subregion sizes (*SR_{meas,i}*, *i=[1,n]*)*.* Additionally, the elements of the ensemble of indicators of image data heterogeneity may be mapped to their corresponding ROI and be graphically represented. By applying the method for heterogeneity analysis to each part (or a plurality of parts) of a studied region, for example, an organ, a comprehensive or more complete overview of the whole region condition may be obtained, and the individual measures of image data heterogeneity provided may be graphically represented to provide a visual insight of the condition and facilitate for example, improved localization of possibly problematic areas.

Proposed embodiments may also leverage the insight that the heterogeneity analysis may not only result in a quantification of the heterogeneity but may also be a tool to estimate a condition of a ROI based on a given reference measure of image data heterogeneity. These embodiments have in common that the step of analyzing the ROI image data heterogeneity may further comprise receiving a reference measure of image data heterogeneity (*D_{ref}*)*.*

To estimate a condition based on a given reference measure of image data heterogeneity (*D_{ref}*)*,* in some embodiments, the step of analyzing the ROI image data heterogeneity may further comprise determining from an ensemble of ROI subregion sizes with corresponding measures of image data heterogeneity, the ROI subregion size (*SRₘₐₓ*) whose measure of image data heterogeneity is maximum (*Dₘₐₓ*) and then determine the indicator of image data heterogeneity based on a comparison of the values of *D_{mαx}* and *D_{ref.}.* By comparing these values, the status of a ROI condition may be assessed. For example, if *Dₘₐₓ* is less than *D_{ref} it* may suggest that the part under study is in a "regular condition" (e.g., a healthy condition or a condition that remains unchanged or was improved since a previous assessment). While if *Dₘₐₓ* is greater or equal than *D_{ref} it* may suggest an "irregular condition" (e.g., an unhealthy or deteriorated condition since a previous assessment).

In some embodiments, the step of analyzing the ROI image data heterogeneity may further comprise estimating a ROI subregion size of the DAX image data (*SR_{meas,i}*) whose measure of image data heterogeneity (*D_{meas,i}*) is approximately equal to *D_{ref}.* The indicator of image data heterogeneity is the estimated *SR_{meas,i}*. In this way, the size of the subregion (*SR_{meas,i}*) for which the mentioned condition is met may provide an insight into the status or severity of the condition of the part being analyzed.

In some embodiments, the step of analyzing the ROI image data heterogeneity may further comprise determining an ensemble of ROI subregion sizes of the DAX image data (*SR_{meas,i}*, *i=[1,n]*) whose measure of image data heterogeneity (*D_{meas,i}*,*i=[1,n]*) is greater than *D_{ref}.* The indicator of image data heterogeneity is the ensemble of estimated ROI subregion sizes (*SR_{meas,i}, i=[1,n]*) and/or corresponding measures of image data heterogeneity (*D_{meas,i}*, *i=[1,n]*)*.* This is an alternative way to provide an insight into the status of the condition of a ROI, and possibly its extent, by determining the subregion sizes and corresponding measures of image data heterogeneity that are above a given reference.

According to a second aspect of the invention, there is provided an apparatus for heterogeneity analysis in 3D-DAX comprising a processing unit including:
- an input unit configured to receive 3D X-ray dark-field (DAX) image data and/or 3D X-ray attenuation image data of a subject;
- a segmentation unit configured to:
   segment the X-ray attenuation image data and/or the DAX image data to identify a region of interest (ROI) in the DAX image data;
- a heterogeneity analyzing unit configured to analyze the image data heterogeneity by performing a statistical analysis of the ROI in the DAX image data based on dividing the ROI in the DAX image data in a number *N* of subregions with an associated subregion size (*SRₘₑₐₛ*) and quantifying a corresponding measure of image data heterogeneity (*Dₘₑₐₛ*); and
- an output unit configured to provide an indicator of image data heterogeneity.

As such, the apparatus is capable of performing all previously discussed steps, and may enable the user to have the benefits of the method from the first aspect of the invention.

According to a third aspect of the invention, there is provided a system for 3D X-ray imaging comprising a 3D X-ray acquisition equipment configured to acquire and to provide 3D DAX image data and 3D X-ray attenuation image data of a subject; and an apparatus according to the second aspect of the invention. In this way the system may also provide means to assist a user in the analysis of heterogeneity in 3D-DAX image data of a subject.

In some embodiments, the 3D X-ray acquisition equipment may be a computed tomography, X-ray tomosynthesis, or C-arm X-ray imaging system. These are the most commonly used 3D X-ray acquisition devices and available at many hospitals or other imaging locations.

According to a fourth aspect of the invention, there is provided a computer program element which, when being executed by at least one processing unit, is adapted to cause the processing unit to perform the steps of the method and/or to control the apparatus according to the second aspect of the invention, and/or the system according to the third aspect of the invention.

According to a fifth aspect of the invention, there is provided a computer readable medium having stored the program element.

It is noted that 'subject' in the context of the invention may refer to a dead or living human or animal body or to a plant or a portion of a plant. However, it may also stand for an artificial part like a machine part or an assembly of parts, wherein these parts may be made out of one or more materials like metal, ceramic, or plastic materials, which when phase contrast imaged exhibits signal heterogeneity.

These and other aspects of the present invention will become apparent from and elucidated with reference to the embodiments described hereinafter.

### BRIEF DESCRIPTION OF THE DRAWINGS

In the following drawings:
Fig. 1 shows a flow diagram or a method for heterogeneity analysis in 3D-DAX imaging, as described in conjunction with the presently claimed invention.
Fig. 2 shows a schematic representation of a thorax of a subject. Fig. 2A shows an attenuation image of the thorax of the subject. Fig. 2B shows a slice of a 3D-DAX image of the thorax of the subject with a region of interest divided in subregions. Fig. 2C shows a slice of a 3D-DAX image of the thorax of the subject depicting two regions of interest (*ROI₁* and *ROI₂*)*.*
Fig. 3 shows a block diagram representing a system for 3D X-ray imaging comprising an apparatus for heterogeneity analysis in 3D-DAX imaging, as described in conjunction with the presently claimed invention.

The invention may take form in various components and arrangements of components, and in various processes operations and arrangements of process operations. The drawings are only for the purpose of illustrating the preferred embodiments and are not to be construed as limiting the invention. To better visualize certain features may be omitted or dimensions may be not according to scale. Like or similar components are given the same reference numerals in different figures.

### DETAILED DESCRIPTION OF THE EMBODIMENTS

In this detailed description, various specific details are presented. Embodiments of the present invention can be carried out without these specific details. Furthermore, well-known features, elements and/or steps are not necessarily described in detail for the sake of clarity and conciseness of the present disclosure.

Implementations in accordance with the present disclosure relate to various techniques, methods, schemes and/or solutions pertaining to analyzing signal heterogeneity in three-dimensional X-ray dark-field (3D-DAX) imaging. The inventors have recognized that such analysis may be leveraged to generate a clinical parameter that may act as clinical biomarker to help physicians in their assessments, e.g., to determine a severity of the disease and/or localization of problematic areas. Hence, according to proposed concepts, a number of possible solutions may be implemented separately or jointly. That is, although these possible solutions may be described in the following separately, two or more of these possible solutions may be implemented in one combination or another.

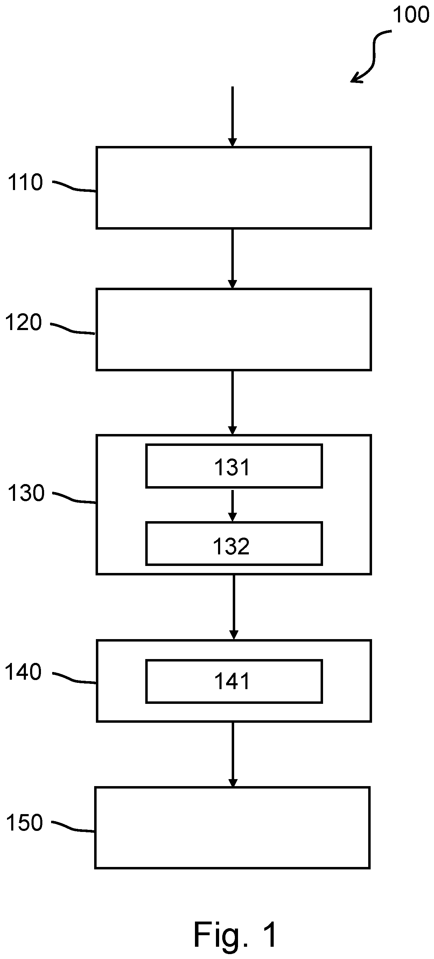

In a first aspect of the invention a method for heterogeneity analysis in 3D-DAX image data is provided and presented in Fig. 1 in its basic steps.

At step 110, also referred to as step (a), an X-ray dark field (DAX) image data and/or X-ray attenuation image data of a subject are received. The DAX and X-ray attenuation image data are 3D imaging data including a plurality of voxels, and each voxel is related to a DAX (sometimes call diffusion) value and attenuation value, respectively. The DAX image and the attenuation image usually are perfectly registered as both images are acquired at the same time and in the same dataset. The perfect registration also means that voxels of the DAX image may be mapped onto voxels of the attenuation image. The image data may be acquired directly from phase contrast imaging the subject or may be obtained from a database.

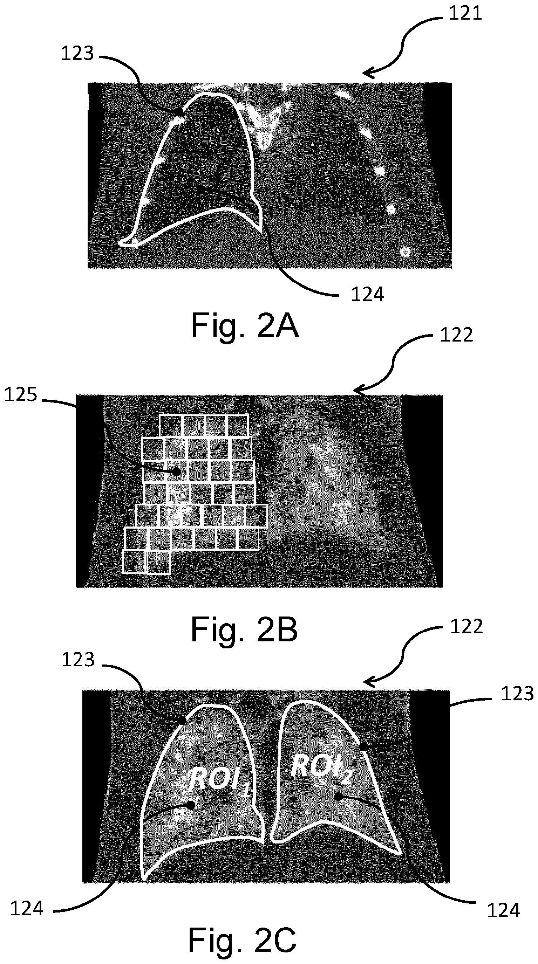

At step 120, also referred to as step (b), the X-ray attenuation image data and/or the DAX image data is segmented to identify a region of interest (ROI) in the DAX image data. Segmentation techniques for attenuation image data are well known to those skilled in the art and will not be further discussed. It is evident to those skilled in the art that those existing concepts may be adapted for DAX image data. By way of example, Fig. 2 shows a schematic representation of a thorax of a subject, in this example a mouse; the representation was adapted from A. Velroyen et al "Grating-based X-ray dark-field computed tomography of living mice", EBioMedicine 2, 10 (2015). In Fig. 2A an attenuation image 121 of the thorax of the patient is shown. Figs. 2B and 2C show a slice of a 3D-DAX image 122 of the thorax of the subject. The segmentation contour 123 delimits the ROI 124 which in this example is a lung. The ROI 124 may be any organ (or part thereof), or structure of the subject capable of being imaged using DAX imaging and exhibiting signal heterogeneity.

At step 130, also referred to as step (c), the heterogeneity of the ROI image data is analyzed by performing a statistical analysis of the ROI 124 in the DAX image data.

The analysis is based, as illustrated in Fig. 2B, on dividing the ROI 124 in the DAX image data in a number N of subregions 125. The subregions 125, that may be also named kernels 125, have an associated subregion size (*SRₘₑₐₛ*)*.* Therefore, the number *N* may be given by the image size divided by the subregion 125 or kernel 125 size. The size of the subregion 125 may be expressed in voxel units or in physical length parameters (e.g., millimeter. Further, the subregion 125 may be of any shape, such as any polygonal prism, spherical, or ellipsoidal shape, and the subregions 125 may also overlap. Fig. 2B shows a schematic representation of the ROI 124 divided into a number *N* of subregions 125 of cubical shape.

Additionally, to ensure that non-ROI voxels are excluded from the analysis, a mask covering the ROI 124 may be first determined such that the subregions 125 (e.g., a defined subregion being a sphere with 10 pixels diameter) lying in the boundary of the ROI 124 are cropped with the mask.

Continuing with the analysis, once the ROI 124 in the DAX image data is divided in subregions 125, a corresponding ROI measure of image data heterogeneity (*Dₘₑₐₛ*) is quantified.

In an embodiment, the measure of image data heterogeneity (*Dₘₑₐₛ*) comprises obtaining a measure of central tendency per subregion *N* of the DAX image data, and subsequently, a measure of statistical dispersion based on the ensemble of measures of central tendency of the DAX image data per subregion *N*. The term 'central tendency' is a standard expression in statistics that could indicate the mode, median, or mean, and that a measure of statistical dispersion may include the variance, standard deviation, or any other parameter related to the quantification of variation. In an exemplary embodiment, the mean dark-field signal per subregion *N* may be calculated and then the variance, or the standard deviation based on the ensemble of mean values per subregion previously computed, may be calculated to obtain a measure of image data heterogeneity (*Dₘₑₐₛ*)*.*

By obtaining a measure of central tendency within each subregion 125, ensures that the statistical dispersion analysis is done on the proper length scale as defined by the subregion size (*SRₘₑₐₛ*)*.* Therefore, the ROI may be characterized by a measure of image data heterogeneity (*Dₘₑₐₛ*) for a given subregion size (*SRₘₑₐₛ*)*.*

Yet, as an additional part of the analysis, a filtering operation (e.g., low-pass filter, gaussian filter) on the ROI 124 may be applied prior to obtaining the measure of statistical dispersion. The measure of central tendency could in this case be considered in fact as a special low-pass filtering operation with a statistical motivation. However, other filtering operations may be applied, for example to exclude certain structures in the ROI 124, for instance, bronchi or larger vessels in the lung, and hence lead to even more accurate and/or sharper results.

Turning back to the method steps, at step 140, also referred to as step (d), an indicator of image data heterogeneity is provided. In an embodiment, the indicator of image data heterogeneity comprises the measure of image data heterogeneity (*Dₘₑₐₛ*) and/or the corresponding subregion size (*SRₘₑₐₛ*)*.*

As mentioned previously, the ROI 124 may be any structure, organ (or part thereof) of the subject exhibiting signal heterogeneity. For example, the ROI may be the entire lung, but also only left or right lung, or even a single lung lobe. The ROIs 124 may also overlap. Fig. 2C schematically illustrates two ROIs representing a right and a left lung, labeled as *ROI₁* and *ROI₂.* Therefore, in an embodiment, the ROI is an ensemble of ROIs (*ROIᵢ, i=[1,n]*)*,* each ROI 124 characterized by a measure of image data heterogeneity (*Dₘₑₐₛ*) for a subregion size (*SRₘₑₐₛ*)*.* Thus, the indicator of image data heterogeneity is an ensemble of the measures of image data heterogeneity (*D_{meas,i}, i=[1,n]*) and/or an ensemble of subregion sizes (*SR_{meas,i}, i=[1,n]*)*,* such that each *ROIᵢ* is related to its corresponding measure of image data heterogeneity (*D_{meas,i}*) and its corresponding subregion size (*SR_{meas,i}*)*.* In other words, there is a mapping between the elements of the ensemble of indicators of image data heterogeneity to their corresponding ROI. In an embodiment, step 140 further comprises this mapping 141, which may be presented using a graphical representation such as a heatmap. The mapping may be visualized 150 in a display screen or in hardcopy form. Additionally, or alternatively, the mapping 141 may be provided as a dataset in the form of a table or any other appropriate format for further processing. For example the measures of image data heterogeneity (*D_{meas,i}, i=[1,n]*) may be visualized as a heatmap when overlayed on their corresponding ROI (*ROIᵢ, i=[1,n]*)*.* By applying the method for heterogeneity analysis previously described to each part (or a plurality of parts) of the studied organ a comprehensive or more complete overview of an organ condition may be obtained, and the individual measures of image data heterogeneity may be graphically represented to provide a visual insight of the condition, and facilitate for example, improved localization of possibly problematic areas. For graphical representation, the ensemble of ROI might contain for each voxel in the organ a ROI, which is centered around the voxel.

Another aspect of the previously described analysis is to obtain an insight on the condition of a ROI in relation to a reference measure of image data heterogeneity (*D_{ref}*)*.* To this end, in some embodiments, step 130, also referred to as step (c), further comprises step 131, also referred to as step (c1), and step 132, also referred to as step (c2).

At step 131, a reference measure of image data heterogeneity (*D_{ref}*) is received. The reference measure of image data heterogeneity may be received from previous studies of the same ROI of the subject under examination, or it may be a general reference measure for a ROI, it may be also received from a database, or received as part of a test or calibration procedure, etc.

In an embodiment, at step 132, the subregion size (*SRₘₐₓ*) whose measure of image data heterogeneity is maximum (*Dₘₐₓ*) is determined from an ensemble of ROI subregion sizes (*SR_{meas,i}, i=[1,n]*) with corresponding measures of image data heterogeneity (*D_{meas,i}, i=[1,n]*)*.* Then, the indicator of image data heterogeneity of step 140 is based on a comparison of the values of *Dₘₐₓ* and *D_{ref}.* For example, a condition indicator may be provided wherein if *Dₘₐₓ* is less than *D_{ref}* it may suggest that the organ or part under study is in a "regular condition" (e.g., a healthy condition or a condition that remains unchanged or was improved since a previous assessment). While if *Dₘₐₓ* is greater or equal than *D_{ref}* it may suggest an "irregular condition" (e.g., an unhealthy or deteriorated condition since a previous assessment) which could be further investigated.

In an alternative embodiment, at step 132, a ROI subregion size of the DAX image data (*SR_{meas,i}*) whose measure of image data heterogeneity (*D_{meas,i}*) is approximately equal to *D_{ref}* is estimated. Here the term 'approximately' means within a range of 10% above or below the stated value, preferably within a range of 5% above or below the stated value, and more preferably within a range of 1% above or below the stated value. Then, the indicator of image data heterogeneity of step 140 is the estimated subregion size (*SR_{meas,i}*) for which the stated condition is met. In this way, the size of the subregion (*SR_{meas,i}*) for which the mentioned condition is met may provide an insight into the status of the condition of the ROI being analyzed (e.g., severity, whether condition is improving or regressing, etc.).

In another embodiment, at step 132, an ensemble of ROI subregion sizes of the DAX image data (*SR_{meas,i, i=[1,n]}*) whose measure of image data heterogeneity (*D_{meas,i i=[1,n]}*) is greater than *D_{ref}* is estimated. Then, the indicator of image data heterogeneity of step 140 is the ensemble of estimated ROI subregion sizes (*SR_{meas,i}, _{i=[1,n]}*) and/or corresponding measures of image data heterogeneity (*D_{meas,i}, _{i=[1,n]}*)*.* By determining the subregion sizes and corresponding measures of image data heterogeneity that are above a given reference (*D_{ref}*)*,* an estimation and extent of the condition of the ROI may be appreciated (e.g., magnitude of the condition, growth rate of the condition, etc.).

Clearly, the implementation of any of the variations of step 132 may be performed manually, semiautomatically, or automatically by any type of algorithm, including computer aided or artificial intelligence-based algorithms. Moreover, the corresponding embodiments may be applied to an ensemble of ROIs for a more in-depth analysis. Also, it is evident that the parameters to be compared (e.g., *Dᵣₑⱼ* versus *Dₘₐₓ* or *D_{meas,i}*) are in a format (i.e., units) in which they can be directly compared, or alternatively, it will be known for the person skilled in the art how to convert them into a compatible format for comparison.

Further, in general, the person skilled in the art will know that the size of the subregion may depend on a number of factors, such as the pixel size of the detector, the X-ray source and location of the object, therefore, the person skilled in the art will also know how to determine the boundary conditions in order for example to define an ensemble of subregion sizes to be analyzed. Furthermore, it is acknowledged that the measures provided are not an absolute indicator of a ROI condition as they may be influenced for example, by noise in the signal.

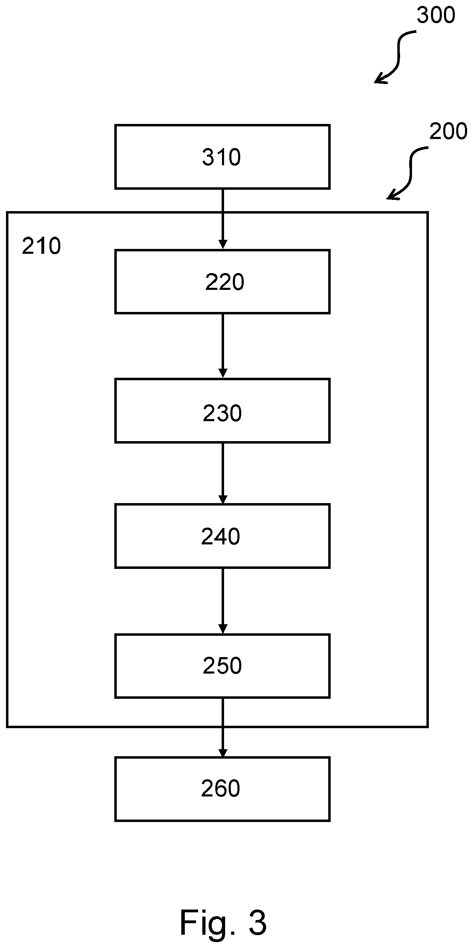

Fig. 3 shows a simplified block diagram 200 of an apparatus for heterogeneity analysis in 3D-DAX imaging.

The apparatus comprises a processing unit 210 including an input unit 220 configured to receive 3D DAX image data and/or 3D X-ray attenuation image data of a subject. The image data may be received from a database or may be acquired directly from a phase contrast X-ray imaging system. For example, the image data may be received from a 3D X-ray acquisition equipment configured to acquire and to provide X-ray DAX image data and X-ray attenuation image data of a subject.

Further, the processing unit 210 includes a segmentation unit 230, a heterogeneity analyzing unit 240, and an output unit 250. The segmentation unit 230 is configured to segment the X-ray attenuation image data and/or the DAX image data to identify a ROI 124 in the DAX image data. The heterogeneity analyzing unit 240 is configured to analyze the ROI image data heterogeneity by performing a statistical analysis of the ROI 124 in the DAX image data based on dividing the ROI 124 in the DAX image data in a number *N* of subregions 125 with an associated subregion size (*SRₘₑₐₛ*) and quantifying a corresponding measure of image of image data heterogeneity (*Dₘₑₐₛ*)*.* The output unit 250 is configured to provide an indicator of image data heterogeneity.

As such, the apparatus whose simplified block diagram is depicted in Fig. 3 may be capable of performing all of the embodiments described previously and hence the apparatus may therefore further comprise a visualization unit 260 configured to display the analyzed data, for example, in a graphical representation.

Additionally, Fig. 3 shows a simplified block diagram 300 of a system for 3D X-ray imaging comprising a 3D X-ray acquisition equipment 310 and an apparatus 200 as previously described. The 3D X-ray acquisition equipment 310 is configured to acquire and to provide 3D DAX image data and 3D X-ray attenuation image data of a subject and may be a computed tomography, X-ray tomosynthesis, or C-arm X-ray imaging system enhanced with a phase contrast X-ray imaging implementation.

In this way, the apparatus 200 and the system 300 may provide means to assist a user in the analysis of heterogeneity in 3D-DAX image data of a subject.

In another embodiment of the present invention, a computer program element is provided, which when being executed by at least one processing unit, is adapted to cause the processing unit to perform the steps of the method 100 and/or to control the apparatus 200 and/or the system 300, previously described.

According to a further embodiment of the present invention, a computer readable medium is presented wherein the computer readable medium has a computer program element stored on it which computer program element is described by the preceding section.

The instructions may be stored on a computer program product. The computer program product may be provided by dedicated hardware as well as hardware capable of executing software in association with appropriate software. When provided by a processor, the functions can be provided by a single dedicated processor, by a single shared processor, or by a plurality of individual processors, some of which can be shared.

For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system, or apparatus or device, or a propagation medium. Examples of a computer-readable medium include a semiconductor or solid state memory, magnetic tape, a removable computer diskette, a random access memory "RAM", a read-only memory "ROM", a rigid magnetic disk and an optical disk. Current examples of optical disks include compact disk ― read only memory "CD-ROM", compact disk ― read/write "CD-R/W", Blu-Ray^{™} and DVD. Examples of a propagation medium are the Internet or other wired or wireless telecommunication systems.

While the invention has been illustrated and described in detail in the drawings and foregoing description, such illustration and description are to be considered illustrative or exemplary and not restrictive; the invention is not limited to the disclosed embodiments.

Variations to the disclosed embodiments can be understood and effected by those skilled in the art in practicing the claimed invention, from a study of the drawings, the disclosure, and the appended claims. It is noted that the various embodiments may be combined to achieve further advantageous effects.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. Method (100) for heterogeneity analysis in 3D X-ray dark-fieldimaging, comprising:
(a) receiving (110) 3D X-ray dark-field (DAX) image data and/or 3D X-ray attenuation image data of a subject;
(b) segmenting (120) the X-ray attenuation image data and/or the DAX image data to identify a region of interest (ROI) in the DAX image data;
(c) analyzing (130) the ROI (124) image data heterogeneity by performing a statistical analysis of the ROI in the DAX image data based on dividing the ROI in the DAX image data in a number *N* of subregions (125) with an associated subregion size (*SRₘₑₐₛ*)*,* and quantifying a corresponding measure of image data heterogeneity (*Dₘₑₐₛ*); and
(d) providing (140) an indicator of image data heterogeneity.

2. Method according to claim 1, wherein quantifying the measure of image data heterogeneity comprises obtaining a measure of central tendency of the DAX image data per subregion *N*, and a measure of statistical dispersion based on the ensemble of measures of central tendency of the DAX image data per subregion *N*.

3. Method according to claims 1-2 wherein the indicator of image data heterogeneity comprises the measure of image data heterogeneity (*Dₘₑₐₛ*) and/or the corresponding subregion size (*SRₘₑₐₛ*)*.*

4. Method according to claims 1-3 wherein the region of interest (ROI) is an ensemble of ROIs (*ROIᵢ, i=[1,n]*)*,* each ROI **characterized by** a measure of image data heterogeneity for a subregion size, and wherein the indicator of image data heterogeneity is an ensemble of measures of image data heterogeneity (*D_{meas,i}, i=[1,n]*) and/or an ensemble of subregion sizes (*SR_{meas,i}*, *i=[1,n]*)*,* and wherein step (d) (140) further comprises mapping (141) the elements of the ensemble of indicators of image data heterogeneity to their corresponding ROI.

5. Method according to claims 1-2, wherein step (c) (130) comprises:
(c1) receiving (131) a reference measure of image data heterogeneity (*D_{ref}*);
(c2) determining (132) from an ensemble of ROI subregion sizes with corresponding measures of image data heterogeneity, the ROI subregion size (*SRₘₐₓ*) whose measure of image data heterogeneity is maximum (*Dₘₐₓ*);
and wherein the indicator of image data heterogeneity is based on a comparison of the values of *Dₘₐₓ* and *D_{ref.}.*

6. Method according to claims 1-2, wherein step (c) (130) comprises:
(c1) receiving (131) a reference measure of image data heterogeneity (*D_{ref}*);
(c2) estimating (132) a ROI subregion size of the DAX image data (*SR_{meas,i}*) whose measure of image data heterogeneity (*D_{meas,i}*) is approximately equal to *Dᵣₑ_{ḟ},*
and wherein the indicator of image data heterogeneity is the estimated *SR_{meas,i}.*

7. Method according to claims 1-2, wherein step (c) (130) comprises:
(c1) receiving (131) a reference measure of image data heterogeneity (*D_{ref}*);
(c2) determining (132) an ensemble of ROI subregion sizes of the DAX image data (*SR_{meas,i}, i=[1,n]*) whose measure of image data heterogeneity (*D_{meas,i}, i=[1,n]*) is greater than *Dᵣₑ_{ḟ},*
and wherein the indicator of image data heterogeneity is the ensemble of estimated ROI subregion sizes (*SR_{meas,i}*, *i=[1,n]*) and/or corresponding measures of image data heterogeneity (*D_{meas,i}, i=[1,n]*)*.*

8. An apparatus (200) for heterogeneity analysis in 3D X-ray dark-field imaging, comprising:
a processing (210) unit including:
- an input unit (220) configured to receive 3D X-ray dark-field (DAX) image data and/or 3D X-ray attenuation image data of a subject;
- a segmentation unit (230) configured to:
segment (123) the X-ray attenuation image data and/or the DAX image data to identify a region of interest (ROI) in the DAX image data;
- a heterogeneity analyzing unit (240) configured to analyze the image data heterogeneity by performing a statistical analysis of the ROI (124) in the DAX image data based on dividing the ROI in the DAX image data in a number *N* of subregions (125) with an associated subregion size (*SRₘₑₐₛ*) and quantifying a corresponding measure of image data heterogeneity (*Dₘₑₐₛ*); and
- an output unit (250) configured to provide an indicator of image data heterogeneity.

9. Apparatus according to claim 8, wherein quantifying the measure of ROI image data heterogeneity comprises obtaining a measure of central tendency of the DAX image data per subregion *N*, and a measure of statistical dispersion based on the ensemble of measures of central tendency of the DAX image data per subregion *N*.

10. Apparatus according to claims 8-9 wherein the indicator of image data heterogeneity comprises the measure of image data heterogeneity (*Dₘₑₐₛ*) and/or the corresponding subregion size (*SRₘₑₐₛ*)*.*

11. Apparatus according to claims 8-10 wherein the region of interest (ROI) is an ensemble of ROIs (*ROIᵢ, i=[1,n]*), each ROI **characterized by** a measure of image data heterogeneity for a subregion size, and wherein the indicator of image data heterogeneity is an ensemble of measures of ROI image data heterogeneity (*D_{meas,i}, i=[1,n]*) and/or an ensemble of subregion sizes (*SR_{meas,i}, i=[1,n]*), and wherein the output unit is further configured to map the elements of the ensemble of indicators of image data heterogeneity to their corresponding ROI.

12. A system (300) for 3D X-ray imaging comprising:
- a 3D X-ray acquisition equipment (310) configured to acquire and to provide 3D X-ray dark-field (DAX) image data and 3D X-ray attenuation image data of a subject; and
- an apparatus (200) comprising a processing unit (210) including:
- an input unit (220) configured to receive 3D X-ray dark-field (DAX) image data and/or 3D X-ray attenuation image data of a subject;
- a segmentation unit (230) configured to:
segment the X-ray attenuation image data and/or the DAX image data to identify a region of interest (ROI) in the DAX image data;
- a heterogeneity analyzing unit (240) configured to analyze the ROI image data heterogeneity by performing a statistical analysis of the ROI in the DAX image data based on dividing the ROI in the DAX image data in a number *N* of subregions with an associated subregion size (*SRₘₑₐₛ*) and quantifying a corresponding measure of image data heterogeneity (*Dₘₑₐₛ*); and
- an output unit (250) configured to provide an indicator of image data heterogeneity.

13. System according to claim 12, wherein the 3D X-ray acquisition equipment is a computed tomography, X-ray tomosynthesis or C-arm X-ray imaging system.

14. A computer program element, which, when being executed by at least one processing unit, is adapted to cause the processing unit to perform the steps of the method as per any one of claims 1-7 and/or to control the apparatus of claims 8-11, and/or the system of claims 12-13.

15. A computer readable medium having stored thereon the program element of claim 14.
